# FASCICULE DE BREVET EUROPEEN

(11) **EP 0 858 834 B1**
(45) Date de publication et mention de la délivrance du brevet: **19.11.2003**
(21) Numéro de dépôt: 98400371.5
(22) Date de dépôt: 17.02.1998
(51) Int. Cl.: B01J 29/04, B01J 23/62, C07C 5/333

(54) **Composition catalytique pour la déshydrogénation d'alcanes en alcènes, sa préparation et son utilisation**
Katalytische Zusammensetzung zur Dehydierung von Alkanen und Alkylenen, und Verfahren zur Herstellung und zur Verwendung der Zusammensetzung
Catalytic composition for the dehydrogenation of alkanes and alkenes, and preparation and use of the same

(30) Priorité: 18.02.1997 FR 9701867
(43) Date de publication de la demande: 19.08.1998
(73) Titulaire: TotalfinaElf France, 92800 Puteaux (FR)
(72) Inventeur: Meriaudeau, Paul, 01160 Pont D'Ain (FR); Nascimento, Pedro, 76600 Le Havre (FR); Sapaly, Gilbert, 69009 Lyon (FR); Naccache, Claude, 69130 Ecully (FR)
(74) Mandataire: Jolly, Jean-Pierre

(56) Documents cités:
- EP-A- 0 351 066
- EP-A- 0 441 430
- EP-A- 0 457 640
- EP-A- 0 530 069
- US-A- 5 108 969
- M RIAUDEAU ET AL: "Platinum indium bimetallic in silicalite: preparation, characterization and use in the vinylcyclohexane transformation" 11TH INTERNATIONAL CONGRESS ON CATALYSIS PART B, 30 juin 1996 - 5 juillet 1996, BALTIMORE, pages 1313-1320, XP002043500

## Description

La présente invention a pour objet des compositions catalytiques pour la déshydrogénation des alcanes en alcènes. L'invention concerne également leur préparation et leur utilisation pour la déshydrogénation d'alcanes en C₂-C₁₅ en oléfines correspondantes et en hydrogène.

Le procédé de déshydrogénation des alcanes en alcènes est utilisé industriellement. Ce procédé consiste à mettre à réagir des alcanes avec une composition catalytique de déshydrogénation. Deux familles de compositions catalytiques sont employées : la première contenant du chrome supporté sur alumine et la seconde contenant du platine supporté sur alumine. On sait également que la conversion en alcènes est d'autant plus difficile que la chaîne carbonée de l'alcane utilisé est plus courte.

De nombreuses compositions catalytiques de déshydrogénation sont décrites dans la littérature. Généralement, les compositions catalytiques contiennent des métaux tels que le platine, le chrome, le gallium, etc... Les métaux sont incorporés à des matériaux formant le support ou la matrice du catalyseur ; par exemple, le matériau peut être une alumine, une aluminosilicate, une silice ou une silicalite.

La conversion des alcanes en alcènes tels que l'éthylène, le propylène, le butène ou l'isobutène, est essentielle, notamment dans le domaine du pétrole pour la valorisation de certaines coupes pétrolières. En particulier, l'isobutène et l'isopentène permettent la préparation du méthyl-tertio-butyl-éther et du tertio-amyl-méthyl-éther, bases utilisées pour augmenter l'indice d'octane des essences.

Toutefois, les combinaisons catalytiques habituellement mises en oeuvre pour cette conversion ont une durée de vie relativement courte nécessitant leur régénération ou leur remplacement pour obtenir des rendements élevés.

A titre d'exemple, les brevets ci-dessous illustrent les tentatives qui ont été faites pour améliorer la durée de vie des compositions catalytiques de déshydrogénation.

Le brevet US 5 147 837 présente une composition catalytique non acide de déshydrogénation comprenant un métal du groupe du platine, un matériau cristallin microporeux dont la structure est de préférence celle d'une zéolite ZSM-5, du titane et en option de l'étain, de l'indium, du plomb ou du thallium.

Le document EP 212 850 présente une composition catalytique de déshydrogénation comprenant un métal du groupe du platine, de la silicalite comme support, mais sans métal alcalin ou alcalino-terreux.

Le document EP 351 066 présente une composition catalytique de déshydrogénation semblable à celle du document EP 212 850 et dont la stabilité est augmentée par l'incorporation d'étain.

Le document EP 623 385 décrit un procédé de préparation d'un catalyseur applicable à la déshydrogénation contenant un support inorganique à base d'oxyde réfractaire tel qu'une alumine, un métal du Groupe VIII tel que le platine, un métal choisi indifféremment dans le groupe constitué par le germanium, l'étain, le plomb, le fer, le titane et le chrome, et éventuellement, de manière préférée, un métalloïde et un halogène.

Dans la publication intitulée "Platinum indium bimetallic in silicalite : preparation, characterization and use in the vinylcyclohexene transformation", pages 1313-1320, XP002043500, rendant compte de l'intervention de M. Mériaudeau et Al, au "11^{TH} International Congress on Catalysis, Part B" qui s'est tenu du 30 juin 1996 au 5 juillet 1996, à Baltimore, Etats-Unis, est décrite la préparation d'un catalyseur contenant de l'indium et du platine sur un support silicalite, et son utilisation dans la transformation du vinylcyclohexène, cette transformation comprenant des réactions de déshydrogénation. Ce catalyseur est dépourvu de germanium et aucune utilisation pour la déhydrogénation des alcanes en alcènes n'est mentionnée.

Le brevet américain n° 5 108 969 concerne une composition catalytique comprenant un support en matériau cristallisé poreux à faible acidité du type zéolithe MCM-22, contenant un métal du groupe VIII, tel que le platine ou le palladium, et pouvant aussi contenir un métal tel que l'étain, l'indium, le thallium; en outre, le support peut être combiné à un liant composé d'oxyde de silicium, de germanium ou de titane. Cette composition est utilisée pour la conversion des alcanes en C₂-C₁₂ en oléfines et/ou aromatiques. Elle a donc un support qui est (faiblement) acide et lorsqu'elle contient du germanium, ce dernier n'a pas de fonction catalytique car il se substitue au silicium dans le réseau de la zéolithe.

Les procédés mettant en oeuvre les compositions catalytiques des documents cités ci-dessus se révèlent peu viables économiquement; en effet, ces compositions catalytiques se désactivent rapidement au cours de leur utilisation, surtout en l'absence d'hydrogène, et présentent donc une stabilité et une sélectivité insuffisantes.

Dans le but d'améliorer les propriétés catalytiques des compositions de l'art antérieur, la Demanderesse a constaté de manière surprenante que le choix du support catalytique et des métaux entrant dans ces compositions jouait un rôle essentiel. En particulier, la Demanderesse a mis en évidence qu'il était particulièrement judicieux de choisir, parmi tous les supports possibles, un support non acide et en particulier un support de type silicalite et de choisir, outre un métal du groupe du platine, deux autres métaux judicieusement sélectionnés : un composé du germanium pour lui conférer une bonne stabilité, et un composé d'étain ou d'indium pour lui conférer une bonne sélectivité. Cette nouvelle démarche est tout à fait innovante puisque l'art antérieur n'évoque aucune sélection des métaux qu'il énumère et se contente de les utiliser au même titre les uns que les autres, sans distinction dans leur rôle catalytique (se reporter en particulier au document EP 623 385).

L'objet de la présente invention est par conséquent une composition catalytique pour la déshydrogénation des alcanes en alcènes et hydrogène, caractérisée en ce qu'elle comprend un support microporeux non acide de type silicalite, exclusivement constitué de silicium, un métal du groupe du platine, un composé à base de germanium et un composé à base soit d'étain, soit d'indium et en ce que, dans le réseau de la silicalite, le silicium n'est pas substitué par le germanium.

La présente invention a également pour objet un procédé de préparation de cette composition catalytique, améliorant encore sensiblement ses qualités.

Un autre objet de l'invention est l'utilisation de la composition catalytique selon l'invention dans un procédé perfectionné pour déshydrogéner un alcane en alcène, selon lequel la sélectivité de la réaction est accrue et l'activité du catalyseur est remarquablement stable au cours de la réaction, tout en assurant des vitesses réactionnelles élevées. Ainsi, grâce à l'utilisation de la nouvelle composition catalytique, le procédé de déshydrogénation ne nécessite pas obligatoirement d'apport d'hydrogène exogène pour maintenir son efficacité.

Selon l'invention, le support catalytique est une silicalite qui est un matériau microporeux non acide exclusivement constitué de silicium contrairement aux autres supports qui contiennent du silicium et de l'aluminium. La silicalite peut exister sous différentes formes structurales selon le mode de préparation choisi. La préparation de la silicalite 1 est décrite dans le brevet US 4 061 724, sa structure est celle d'une M.F.I. (voir "Atlas of zeolite structure types" publié par Butterworth Heinemann, 3ème édition). Une autre forme, la silicalite 2, est décrite dans le journal "Nature", vol. 280, pages 664-665 (1979) de D.M. Bibby, N.B. Milestone et L.P. Aldridge.

Une source commerciale appropriée pour la préparation de la silicalite est le produit LUDOX fabriqué par la société Du Pont.

Le métal du groupe du platine peut être le platine et la source de platine est de préférence choisie parmi l'acide chloroplatinique de formule PtH₂Cl₆ et les sels de platine solubles tels que l'hydroxyde de tétramine de platine de formule Pt(NH₃)₄(OH)₂, et le chlorure de tétramine de platine de formule Pt(NH₃)₄Cl₂.

La teneur de la composition catalytique selon l'invention en métal du groupe du platine est de préférence comprise entre 0,01 et 5 % en poids et en particulier entre 0,01 et 2 % en poids.

La teneur de la composition catalytique selon l'invention en germanium est de préférence comprise entre 0,01 et 5 % en poids et en particulier entre 0,01 et 2 % en poids.

La source de germanium peut être choisie parmi les sels de germanium solubles dans l'eau. De préférence, la source de germanium est le tétraéthoxyde de germanium de formule Ge(OC₂H₅)₄.

Une caractéristique importante de la composition catalytique selon l'invention est que le germanium qu'elle contient a une fonction catalytique, notamment, parce qu'il ne se subsitue pas au silicium dans le réseau de la silicate, cette dernière restant donc exclusivement constituée de silicium.

La teneur de la composition catalytique selon l'invention en étain est de préférence comprise entre 0,01 et 10 % en poids, et en particulier entre 0,01 et 2 % en poids.

La teneur en indium est de préférence comprise entre 0,01 et 10 % en poids, et en particulier entre 0,01 et 2 % en poids.

La source d'indium peut être choisie parmi les sels d'indium solubles dans l'eau tels que le nitrate d'indium, le chlorure d'indium et le sulfate d'indium. De préférence, la source d'indium est le nitrate d'indium de formule In(NO₃)₃.

En présence de la composition catalytique selon l'invention, la conversion et la sélectivité de la réaction de déshydrogénation des alcanes en alcènes sont très élevées. Notamment, dans le cas de la préparation d'isobutène à partir d'isobutane, la conversion et la sélectivité sont respectivement de l'ordre de 40 % et de 98 %, conduisant à un rendement global de l'ordre de 39 %.

En outre, la composition catalytique selon l'invention présente l'avantage de demeurer très performante, même après plusieurs cycles d'utilisation et de régénération.

La composition catalytique peut être préparée selon différentes méthodes connues. Toutefois, un procédé perfectionné a été développé, permettant d'obtenir des compositions catalytiques stables au cours de la réaction de conversion des alcanes en alcènes, tout en augmentant la sélectivité de cette réaction.

Le procédé selon l'invention comprend les étapes suivantes:
(1) formation d'un mélange homogène à partir du support microporeux non-acide de type silicalite, d'un composé à base de germanium et d'un composé à base d'indium ou d'étain. en présence d'un agent structurant;
(2) après cristallisation du mélange homogène à pression autogène et à une température comprise entre 25 et 190°C, filtration et lavage à l'eau du matériau solide obtenu, puis séchage;
(3) calcination du matériau séché, sous atmosphère inerte puis en présence d'oxygène ou d'air, à une température comprise entre 400 et 700°C;
(4) imprégnation du matériau calciné refroidi avec une solution d'une source d'un métal du groupe du platine, sous agitation constante, à une température comprise entre 15 et 95°C ;
(5) évaporation de la phase liquide;
(6) activation du solide récupéré par (i) traitement sous oxygène ou air à une température croissante de 15 à 320°C et (ii) traitement sous hydrogène à une température croissante de 320 à 550°C; et,
(7) refroidissement du catalyseur obtenu.

La cristallisation du mélange dépend d'un certain nombre de facteurs, notamment de la température. Par exemple, si la température est de l'ordre de 170°C, on laissera de préférence la cristallisation s'opérer pendant 1 à 4 jours.

L'étape d'imprégnation (4) s'effectue avantageusement pendant 12 heures; un acide minéral peut être ajouté à la solution, par exemple l'acide nitrique, pour faciliter la dispersion du métal du groupe du platine. De préférence, l'accroissement de la température dans l'étape d'activation (6) est de 0,2°C par minute pour l'oxydation puis de 1°C par minute pour la réduction.

L'agent structurant peut être choisi parmi les amines telles que la diéthylamine, le diamino-1,6-hexane, les alcanolamines telles que la diéthanolamine, les sels d'ammonium tels que le bromure de tétrapropylammonium (TPABr) ou l'hydroxyde de tétrapropylammonium (TPAOH), et les composés similaires.

Le procédé selon l'invention peut comprendre une étape supplémentaire au cours de laquelle on ajoute au catalyseur obtenu une matrice ou un liant, tel que de l'argile, du kaolin, de la silice et/ou un oxyde de métal du type aluminium, titane, zirconium, silicium ou germanium, ou des mélanges d'oxydes de ces métaux faiblement acides ou non acides, puis on lui donne une forme adaptée à la technologie des réacteurs dans lequels il est destiné à être utilisé.

Généralement, le procédé de conversion des alcanes en alcènes en présence d'une composition catalytique selon l'invention est mis en oeuvre à des températures comprises entre 300 et 800°C, à une pression comprise entre 10³ Pa et 10⁶ Pa et à un débit massique de la charge par rapport à la masse de catalyseur (pph) de 1 à 50 h⁻¹. De préférence, lors de la conversion d'isobutane en isobutène, on utilise un gaz inerte et les températures sont comprises entre 500°C et 550°C et la pression est de l'ordre de 10⁵ Pa.

Puisque la réaction de déshydrogénation est endothermique et est limitée par l'équilibre thermodynamique, le procédé est habituellement conduit à haute température et basse pression pour obtenir des taux élevés de conversion. Sous ces conditions sévères, il est habituellement difficile de maintenir une haute activité et une bonne sélectivité sur de longues périodes de temps, parce que les réactions parasites, telles que l'aromatisation, le craquage, l'isomérisation ou la formation de coke, se développent. Toutefois, si les conditions précitées de température et de pression sont respectées, l'activité et la stabilité des nouvelles compositions catalytiques sont améliorées et la sélectivité de production d'alcènes à partir d'alcanes est optimisée.

Lors de la mise en oeuvre du procédé de conversion, on peut également utiliser un diluant tel que l'hydrogène, la vapeur d'eau, un gaz inerte (par exemple l'azote), le méthane, etc., pour diluer la charge d'alcanes. Toutefois, de préférence, la conversion s'effectue en l'absence de diluant.

La conversion en présence des compositions catalytiques de l'invention peut s'effectuer sans dommage malgré l'absence d'H₂ exogène. Toutefois, si l'on souhaite améliorer la stabilité du catalyseur, la réaction est réalisée en présence d'H₂ exogène dans le milieu réactionnel.

L'invention est illustrée ci-après par des exemples de préparation et des tests comparatifs avec les catalyseurs de l'art antérieur et selon l'invention.

### EXEMPLES

Les exemples 1, 2, 3 et 4 représentent des méthodes de préparation de compositions catalytiques selon l'art antérieur. Les exemples 5 et 6 donnés ci-dessous illustrent des méthodes de préparation de la composition catalytique selon la présente invention. L'exemple 7 concerne la mise en forme du catalyseur selon l'invention par élaboration d'extrudés à partir de poudre du catalyseur.

La structure observée aux rayons X du support de chaque catalyseur préparé est celle de la silicalite 1 précisée dans la description.

### Exemple 1

Une composition catalytique A, de formule Pt sur silicalite, a été préparée selon le mode de préparation décrit à l'exemple 1 du document EP 212 850. La composition catalytique préparée contient 0,5% en poids de Pt.

### Exemple 2

Une composition catalytique B, de formule Pt-Sn sur silicalite, a été préparée selon le mode de préparation décrit du document EP 351 066. La composition catalytique préparée contient 0,5% en poids de Pt et 0,8% en poids de Sn.

### Exemple 3

Une composition catalytique C, de formule Pt-Sn sur silicalite a été préparée selon le mode de préparation décrit dans le brevet EP n°351 066. La composition catalytique préparée contient 0,5% en poids de Pt et 0,5% en poids de Sn.

### Exemple 4

Une composition catalytique D, de formule Pt-Ge sur silicalite, a été préparée selon la méthode suivante:
a) 20 g de silicate de sodium sont mis en solution dans 50 g d'eau à laquelle on ajoute ensuite 3 g de TPABr (bromure de tétrapropylammonium),
b) on ajoute à cette solution maintenue sous agitation jusqu'à homogénéisation 30 g d'eau contenant 1,9 g d'H₂SO₄ (solution 1N) et 0,3 g de Ge(OC₂H₅)₄ (tétraéthoxyde de germanium),
c) le mélange homogénéisé est transféré dans un autoclave qui est ensuite chauffé à 175 °C sous pression autogène pendant 3 jours,
d) après lavage et séchage, le solide est calciné sous azote à une température augmentant de 0,2°C par min et allant de 25°C à 550°C, puis placé sous O₂ à cette température de 550°C pendant 12 heures; le solide obtenu contient 1,5% en poids de germanium et la structure observée aux rayons X est celle de la silicalite 1,
e) 10 g du solide sont ensuite ajoutés dans une solution de 100 g d'eau contenant 76 mg de Pt(NH₃)₄(OH)₂,
f) après 24 heures sous agitation à 75°C, l'eau est évaporée et le solide est séché à 80°C,
g) l'activation du catalyseur obtenu s'effectue sous courant d'O₂ à une température progressant de 0,2°C par min et allant de 25 à 320°C, puis pendant 8 heures à cette température,
h) après balayage sous azote, le catalyseur est réduit sous H₂ à une température progressant de 1°C par minute et allant de 320 à 550°C.

La composition catalytique D ainsi obtenue contient 0,5 % en poids de Pt et 1 % en poids de Ge.

### Exemple 5

Une composition catalytique E conforme à l'invention, de formule Pt-Ge-In sur silicalite, a été préparée selon le mode de préparation indiqué à l'exemple 4, à l'exception de la solution de 30 g d'eau de la phase b) qui contient en plus 0,2 g de In(NO₃)₃ ( nitrate d'indium).

La composition catalytique ainsi obtenue contient 0,5 % en poids de Pt, 1 % en poids de Ge et 0,8 % en poids d'In.

### Exemple 6

Une composition catalytique F conforme à l'invention, de formule Pt-Ge-Sn sur silicalite, a été préparée selon le mode de préparation indiqué à l'exemple 4, à l'exception de la solution de 30 g d'eau de la phase b) qui contient 0,33 g de Ge(C₂H₅)₄ et, en plus, 0,09 g de SnSO₄. La composition catalytique ainsi obtenue contient 0,5 % en poids de Pt, 1 % en poids de Ge et 0,5 % en poids de Sn.

### Exemple 7

On mélange 7 g de catalyseur en poudre préparé selon l'exemple 5 ou l'exemple 6, 5,5 g de kaolin, également en poudre, ainsi que 5 ml d'eau distillée. Le mélange est rendu homogène par malaxage et on obtient une pâte qui est passée à travers la filière (1,5 mm de diamètre) d'une seringue-extrudeuse.

On procède ensuite à une calcination des extrudés obtenus dans un four horizontal, avec une montée en température de 1°C/minute jusqu'à 450°C, sous air, puis on maintient la température à cette valeur pendant trois heures.

La masse finale de catalyseur obtenu après refroidissement est de 10 g.

### ESSAIS

Afin d'illustrer les propriétés des compositions catalytiques selon l'invention, les compositions E et F selon l'invention ont été testées et comparées aux compositions A, B, C et D de l'art antérieur préparées selon les exemples 1, 2, 3 et 4, respectivement.

La conversion est définie comme étant la quantité de réactifs (isobutane pour tests 1 et 2, ou propane pour test 3) transformée par quantité de réactifs introduits.

La sélectivité est définie comme étant le nombre de moles d'oléfines formées (isobutène pour tests 1 et 2, ou propène pour test 3) par rapport au nombre de moles de réactifs converties (isobutane ou propane, respectivement).

### Test n°1

Il s'agit de tests de déshydrogénation de l'isobutane en isobutène qui ont été accomplis dans les mêmes conditions pour les cinq combinaisons catalytiques. Les réactions ont été effectuées en présence de 0,05 g de composition catalytique à une température de l'ordre de 530 °C, à une pression de 10⁵ Pa et sans ajout d'H₂ exogène. La pph est égale à 25 h⁻¹.

Les résultats des essais concernant les compositions catalytiques A, B, C, D, E et F sont rassemblés dans le Tableau 1 suivant.

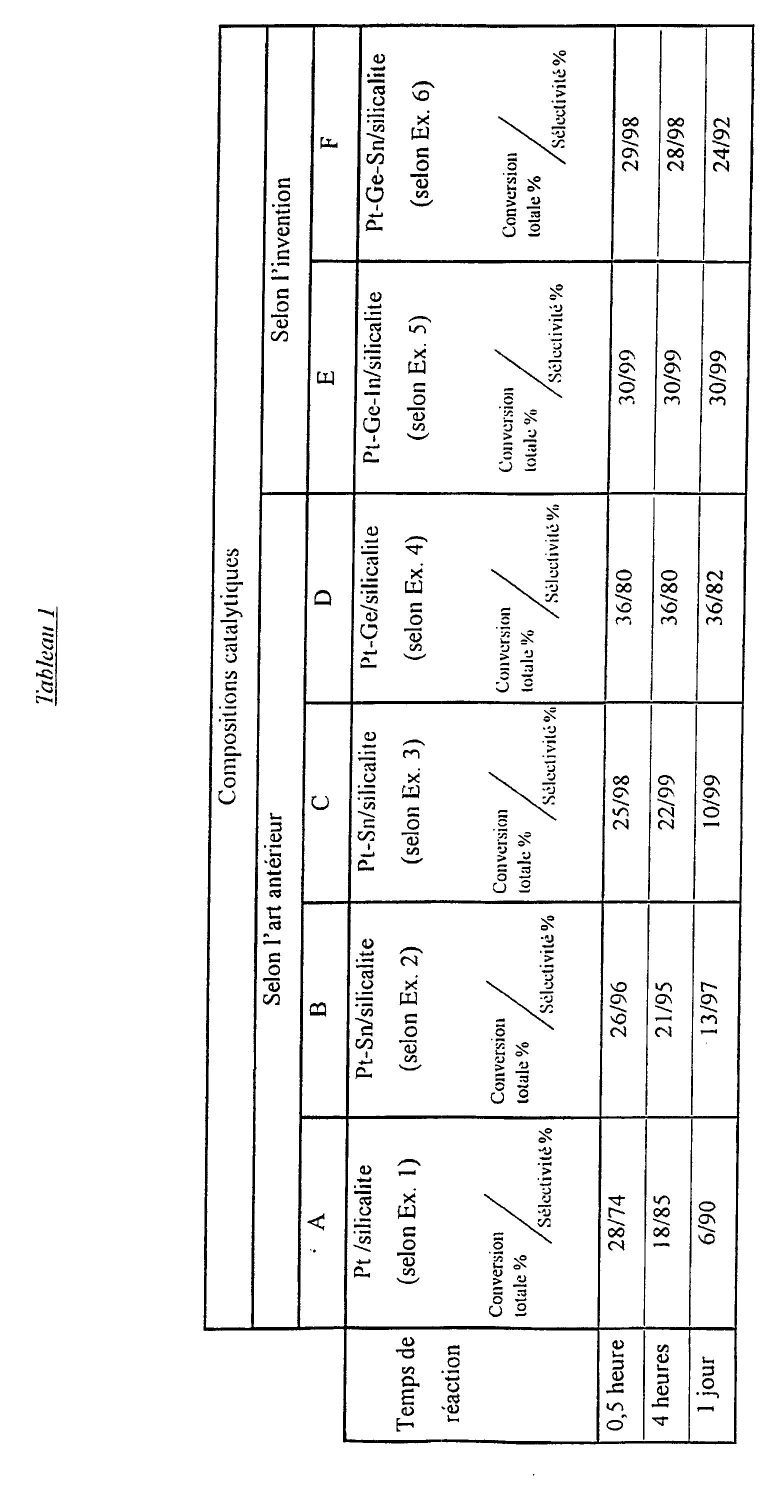

On constate que les résultats obtenus avec les compositions catalytiques E et F (selon l'invention) sont très sensiblement meilleurs à ceux obtenus avec les compositions catalytiques A, B, C et D (selon l'art antérieur) :
- l'addition de germanium dans la composition catalytique améliore considérablement sa stabilité puisque la conversion et la sélectivité restent pratiquement inchangées au cours du temps (comparaison des résultats pour C et F),
- l'addition d'étain dans la composition catalytique améliore considérablement sa sélectivité (comparaison des résultats pour D et F),
- l'addition d'indium dans la composition catalytique améliore considérablement sa sélectivité (comparaison des résultats pour D et E).

En outre, la conversion et la sélectivité des compositions catalytiques selon l'invention demeurent très élevées au-delà de 24 heures et traduisent une activité et une stabilité remarquables et inégalées par rapport aux compositions catalytiques de l'art antérieur (comparaison des résultats de la colonne A avec ceux des colonnes E et F, et comparaison des résultats des colonnes B et F).

### Test n°2

Il s'agit d'autres tests de déshydrogénation de l'isobutane, de plus longue durée, qui ont été effectués avec une composition catalytique Pt-Ge-In/silicalite préparée selon l'exemple 5.

Les réactions ont été effectuées en présence de 0,05 g de composition catalytique, à une pression de 10⁵ Pa et sans ajout d'H₂ exogène. La pph était égale à 26 h⁻¹ dans tous les cas.

Dans un premier temps, la composition catalytique a été testée pendant 7 jours à une température de l'ordre de 530°C.

Puis, dans un second temps, la composition catalytique, après régénération sous H₂ pendant 16 heures à 530°C, a effectué un cycle de travail de 2 jours à une température de l'ordre de 530°C.

Finalement, dans un troisième temps, cette même composition catalytique a été chauffée sous H₂ pendant 6 heures, avant d'être testée pendant 7 jours à une température de l'ordre de 550°C.

Les résultats sont donnés dans le Tableau 2 suivant.

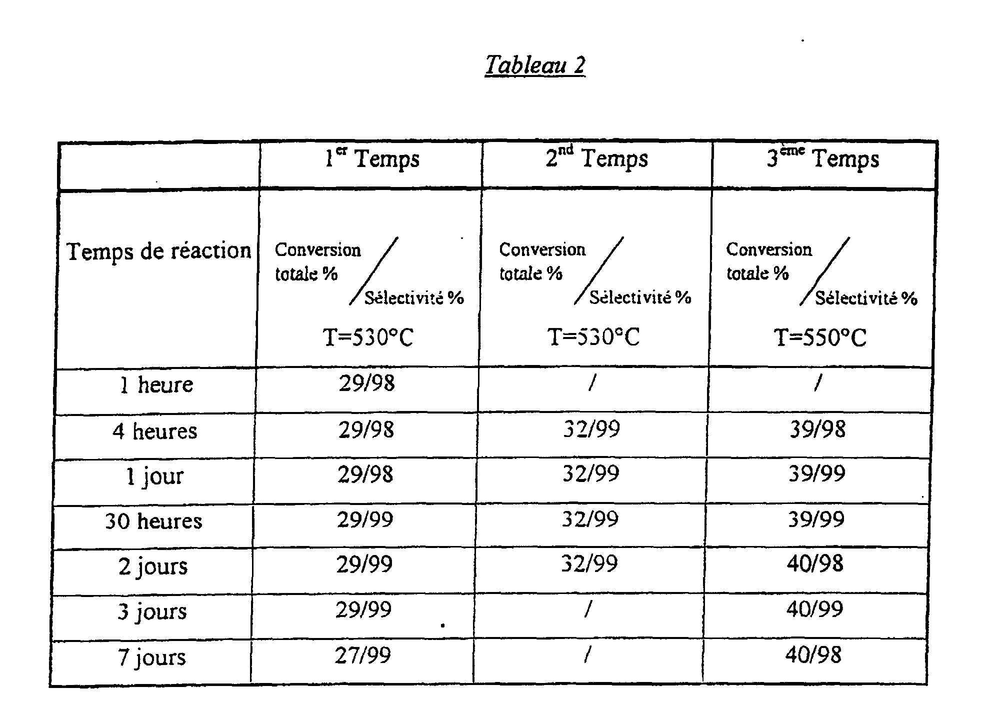

On constate que la conversion et la sélectivité de la composition catalytique selon l'invention selon l'exemple 5 lors de la réaction de déshydrogénation demeurent très élevées sur des périodes de 7 jours, même après régénération.

### Test n°3 (test n'entrant pas dans le cadre de la présente invention).

Il s'agit de tests de déshydrogénation du propane en propène qui ont été effectués en utilisant, comme référence, la composition catalytique Pt sur silicalite de l'art antérieur préparée selon l'exemple 1 et, comme composition catalytique selon l'invention, la composition Pt-Ge-In sur silicalite de l'exemple 5. Les réactions ont été effectuées en présence de 0,05 g de composition catalytique à une température de l'ordre de 550 °C, à une pression de 10⁵ Pa, sans ajout d'H₂ exogène. La pph était égale à 13 h⁻¹.

Les résultats sont donnés dans le Tableau 3 suivant.

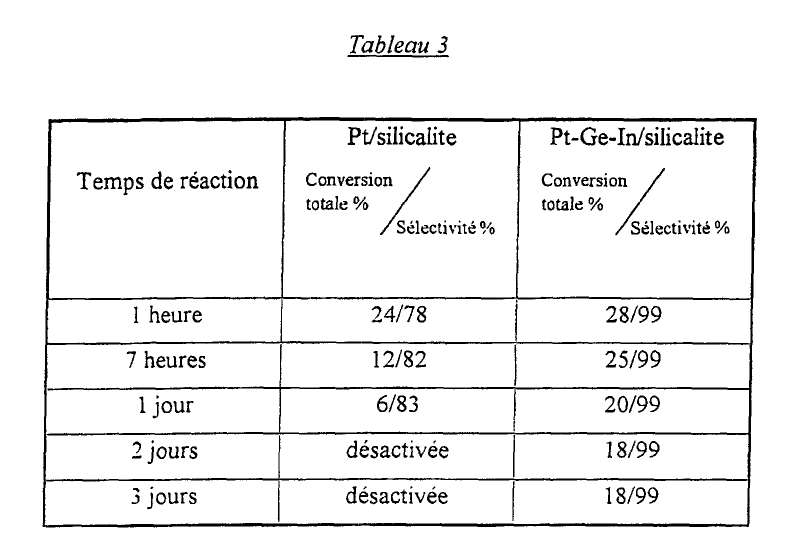

On constate que l'addition de germanium et d'indium dans la composition catalytique améliorent sa stabilité et très nettement sa sélectivité qui reste en outre inchangée au cours du temps.

De plus. la conversion et la sélectivité des compositions catalytiques selon l'invention demeurent très élevées au-delà de 3 jours et traduisent une activité et une stabilité remarquables et inégalées par rapport aux compositions catalytiques de l'art antérieur.

## Revendications

1. Composition catalytique pour la déshydrogénation des alcanes en alcènes et hydrogène, **caractérisée en ce qu'**elle comprend un support microporeux non acide de type silicalite, exclusivement constitué de silicium, un métal du groupe du platine, un composé à base de germanium et un composé à base soit d'étain, soit d'indium, et **en ce que**, dans le réseau de la silicalite, le silicium n'est pas substitué par le germanium.

2. Composition catalytique selon la revendication 1, **caractérisée en ce qu'**elle comprend de 0,01 à 5 % en poids du métal du groupe du platine.

3. Composition catalytique selon la revendication 2, **caractérisée en ce qu'**elle comprend de 0,01 à 2 % en poids du métal du groupe du platine.

4. Composition catalytique selon l'une des revendications précédentes, **caractérisée en ce qu'**elle comprend de 0,01 à 5 % en poids de germanium.

5. Composition catalytique selon la revendication précédente, **caractérisée en ce qu'**elle comprend de 0,01 à 2 % en poids de germanium.

6. Composition catalytique selon l'une des revendications précédentes, **caractérisée en ce qu'**elle comprend de 0,01 à 10 % en poids d'étain.

7. Composition catalytique selon la revendication précédente, **caractérisée en ce qu'**elle comprend de 0,01 à 2 % en poids d'étain.

8. Composition catalytique selon l'une des revendications précédentes, **caractérisée en ce qu'**elle comprend de 0,01 à 10 % en poids d'indium.

9. Composition catalytique selon la revendication précédente, **caractérisée en ce qu'**elle comprend de 0,01 à 2 % en poids en poids d'indium.

10. Procédé de préparation de la composition catalytique selon l'une des revendications précédentes, **caractérisé en ce qu'**il comprend les étapes suivantes:
(1) formation d'un mélange homogène à partir du support microporeux non-acide de type silicalite, d'un composé à base de germanium et d'un composé à base d'indium ou d'étain, en présence d'un agent structurant;
(2) après cristallisation du mélange homogène à pression autogène et à une température comprise entre 25 et 190 °C, filtration et lavage à l'eau du matériau solide ainsi obtenu, et séchage;
(3) calcination du matériau séché, sous atmosphère inerte puis en présence d'oxygène ou d'air. à une température comprise entre 400 et 700 °C;
(4) imprégnation du matériau calciné refroidi avec une solution d'une source d'un métal du groupe du platine, sous agitation constante, à une température comprise entre 15 et 95 °C ;
(5) évaporation de la phase liquide;
(6) activation du solide récupéré par (i) traitement sous oxygène ou air à une température croissante de 15 à 320 °C et, (ii) traitement sous hydrogène à une température croissante de 320 à 550°C; et,
(7) refroidissement du catalyseur obtenu.

11. Procédé selon la revendication 10, **caractérisé en ce qu'**il comprend une étape supplémentaire dans laquelle on ajoute au catalyseur obtenu précédemment une matrice ou un liant, tel que de l'argile, du kaolin, de la silice et/ou un oxyde de métal du type aluminium, titane, zirconium, silicium ou germanium, ou des mélanges d'oxydes de ces métaux faiblement acides ou non acides.

12. Procédé selon la revendication précédente, **caractérisé en ce que** la source de métal du groupe du platine est choisie dans le groupe constitué par l'acide chloroplatinique PtH₂Cl₆ et les sels de platine solubles tels que Pt(NH₃)₄(OH)₂ et Pt(NH₃)₄Cl₂.

13. Utilisation d'une composition catalytique selon l'une des revendications 1 à 9 ou susceptible d'être préparée par un procédé selon l'une des revendications 10 à 12, pour la déshydrogénation des alcanes en alcènes et hydrogène.

14. Utilisation selon la revendication 13, **caractérisée en ce que** la déshydrogénation s'effectue à une température comprise entre 300 et 800 °C, à un débit massique de la charge par rapport à la masse de catalyseur (pph) compris entre 1 et 50 h⁻¹, et à une pression comprise entre 10³ et 10⁶ Pa.

15. Utilisation selon l'une des revendications 13 et 14, **caractérisée en ce que** les alcanes sont l'isobutane et les alcènes l'isobutène, et **en ce que** la déshydrogénation est effectuée à une température comprise entre 500 et 550 °C et à une pression de 10⁵ Pa, en présence d'un gaz inerte.

16. Utilisation selon l'une des revendications 13 à 15, **caractérisée en ce que** la déshydrogénation s'effectue en présence d'hydrogène exogène.

## Patentansprüche

1. Katalytische Zusammensetzung zur Dehydrierung von Alkanen zu Alkenen und Wasserstoff, **dadurch gekennzeichnet, daß** sie einen mikroporösen, nicht sauren Träger vom Typ eines ausschließlich aus Silicium aufgebauten Silicalits, ein Metall der Platingruppe, eine Verbindung auf der Basis von Geramanium und eine Verbindung auf der Basis von Zinn oder Indium umfaßt, und daß im Silicalit-Netzwerk das Silicium nicht durch das Germanium substituiert ist.

2. Katalytische Zusammensetzung gemäß Anspruch 1, **dadurch gekennzeichnet, daß** sie 0,01 bis 5 Gew.-% des Metalls der Platingruppe umfaßt.

3. Katalytische Zusammensetzung gemäß Anspruch 2, **dadurch gekennzeichnet, daß** sie 0,01 bis 2 Gew.-% des Metalls der Platingruppe umfaßt.

4. Katalytische Zusammensetzung gemäß einem der vorangehenden Ansprüche, **dadurch gekennzeichnet, daß** sie 0,01 bis 5 Gew.-% Germanium umfaßt.

5. Katalytische Zusammensetzung gemäß dem vorangehenden Anspruch, **dadurch gekennzeichnet, daß** sie 0,01 bis 2 Gew.-% Germanium umfaßt.

6. Katalytische Zusammensetzung gemäß einem der vorangehenden Ansprüche, **dadurch gekennzeichnet, daß** sie 0,01 bis 10 Gew.-% Zinn umfaßt.

7. Katalytische Zusammensetzung gemäß dem vorangehenden Anspruch, **dadurch gekennzeichnet, daß** sie 0,01 bis 2 Gew.-% Zinn umfaßt.

8. Katalytische Zusammensetzung gemäß einem der vorangehenden Ansprüche, **dadurch gekennzeichnet, daß** sie 0,01 bis 10 Gew.-% Indium umfaßt.

9. Katalytische Zusammensetzung gemäß dem vorangehenden Anspruch, **dadurch gekennzeichnet, daß** sie 0,01 bis 2 Gew.-% Indium umfaßt.

10. Verfahren zur Herstellung der katalytischen Zusammensetzung gemäß einem der vorangehenden Ansprüche, **dadurch gekennzeichnet, daß** es die folgenden Schritte umfaßt:
(1) Bildung einer homogenen Mischung, ausgehend vom mikroporösen, nicht sauren Träger vom Silicalit-Typ, einer Verbindung auf der Basis von Germanium und einer Verbindung auf der Basis von Indium oder Zinn, in Gegenwart eines Strukturmittels;
(2) nach der Kristallisation der homogenen Mischung bei einem selbsterzeugenden Druck und einer Temperatur von zwischen einschließlich 25 und 190°C, Filtrierung und Waschen des so erhaltenen, festen Materials, sowie Trocknung;
(3) Kalzinierung des getrockneten Materials unter einer inerten Atmosphäre, dann in Gegenwart von Sauerstoff oder Luft, bei einer Temperatur von zwischen einschließlich 400 und 700°C;
(4) Imprägnierung des kalzinierten, abgekühlten Materials mit einer Lösung einer Quelle eines Metalls der Platingruppe unter konstanter Bewegung bei einer Temperatur von zwischen einschließlich 15 und 95°C;
(5) Verdampfung der flüssigen Phase;
(6) Aktivierung des wiedergewonnenen Feststoffs durch (i) Behandlung unter Sauerstoff oder Luft bei einer ansteigenden Temperatur von 15 bis 320°C und (ii) Behandlung unter Wasserstoff bei einer ansteigenden Temperatur von 320° bis 550°C; und
(7) Abkühlung des erhaltenen Katalysators.

11. Verfahren gemäß Anspruch 10, **dadurch gekennzeichnet, daß** es einen zusätzlichen Schritt umfaßt, in dem zu dem zuvor erhaltenen Katalysator eine Matrix oder ein Bindemittel wie Ton, Kaolin, Kieselerde und/oder ein Oxid eines Metalls vom Typ Aluminium, Titan, Zirkonium, Silicium oder Germanium oder leicht saure oder nicht-saure Mischungen von Oxiden dieser Metalle hinzugefügt wird.

12. Verfahren gemäß dem vorangehenden Anspruch, **dadurch gekennzeichnet, daß** die Quelle des Metalls der Platingruppe aus der Gruppe ausgewählt wird, die sich aus der Chlorplatinsäure PtH₂Cl₆ und löslichen Platinsalzen wie Pt(NH₃)₄(OH)₂ und Pt(NH₃)₄Cl₂ zusammensetzt.

13. Verwendung einer katalytischen Zusammensetzung gemäß einem der Ansprüche 1 bis 9 oder einer solchen, die im Stande ist, durch ein Verfahren gemäß einem der Ansprüche 10 bis 12 hergestellt zu werden, zur Dehydrierung von Alkanen zu Alkenen und Wasserstoff.

14. Verwendung gemäß Anspruch 13, **dadurch gekennzeichnet, daß** die Dehydrierung bei einer Temperatur von zwischen einschließlich 300 und 800°C bei einem Massedurchsatz der Beladung im Verhältnis zur Katalysatormasse (pph) von zwischen einschließlich 1 und 50 h⁻¹ und bei einem Druck von zwischen einschließlich 10³ bis 10⁶ Pa bewirkt wird.

15. Verwendung gemäß einem der Ansprüche 13 und 14, **dadurch gekennzeichnet, daß** die Alkane Isobutan und die Alkene Isobuten sind, und daß die Dehydrierung bei einer Temperatur von zwischen einschließlich 500 und 550°C und bei einem Druck von 10⁵ Pa in Gegenwart eines inerten Gases bewirkt wird.

16. Verwendung gemäß einem der Ansprüche 13 bis 15, **dadurch gekennzeichnet, daß** die Dehydrierung in Gegenwart von exogenem Wasserstoff bewirkt wird.

## Claims

1. A catalytic composition for the dehydrogenation of alkanes to alkenes and hydrogen, **characterised in that** it comprises a non-acid microporous support of silicalite type, exclusively composed of silicon, a metal of the platinum group, a germanium-based compound and a compound based either on tin or indium, and **in that** in the silicalite network the silicon is not substituted by germanium.

2. A catalytic composition according to claim 1, **characterised in that** it comprises 0.01 to 5 % by weight of the metal from the platinum group.

3. A catalytic composition according to claim 2, **characterised in that** it comprises 0.01 to 2 % by weight of the metal from the platinum group.

4. A catalytic composition according to any one of the preceding claims, **characterised in that** it comprises 0.01 to 5 % by weight of germanium.

5. A catalytic composition according to the preceding claim, **characterised in that** it comprises 0.01 to 2 % by weight of germanium.

6. A catalytic composition according to any one of the preceding claims, **characterised in that** it comprises 0.01 to 10 % by weight of tin.

7. A catalytic composition according to the preceding claim, **characterised in that** it comprises 0.01 to 2 % by weight of tin.

8. A catalytic composition according to any one of the preceding claims, **characterised in that** it comprises 0.01 to 10 % by weight of indium.

9. A catalytic composition according to the preceding claim, **characterised in that** it comprises 0.01 to 2 % by weight of indium.

10. A method of preparing the catalytic composition according to any one of the preceding claims, **characterised in that** it comprises the following steps:
(1) forming a homogeneous mixture from the non-acid microporous support of silicalite type, from a germanium-based compound and from a compound based on tin or indium, in the presence of a structuring agent;
(2) after crystallisation of the homogeneous mixture at autogenous pressure and at a temperature of between 25 and 190°C, filtration and washing with water of the resultant solid material, and drying;
(3) calcination of the dried material, under an inert atmosphere then in the presence of oxygen or air, at a temperature of between 400 and 700°C;
(4) impregnation of the calcined material cooled with a solution of a source of a metal of the platinum group, with constant stirring, at a temperature of between 15 and 95°C;
(5) evaporation of the liquid phase;
(6) activation of the recovered solid by (i) treatment under oxygen or air at a temperature increasing from 15 to 320°C and, (ii) treatment under hydrogen at a temperature increasing from 320 to 550°C, and
(7) cooling of the catalyst obtained.

11. A method according to claim 10, **characterised in that** it comprises an additional step, in which there is added to the previously obtained catalyst a matrix or binder, such as clay, kaolin, silica and/or a metal oxide of aluminium, titanium, zirconium, silicon or germanium type, or weakly acid or non-acid mixtures of oxides of these metals.

12. A method according to the preceding claim, **characterised in that** the source of the platinum group metal is chosen from the group comprising chloroplatinic acid PtH₂Cl₆ and soluble platinum salts, such as Pt(NH₃)₄(OH)₂ and Pt(NH₃)₄Cl₂.

13. Use of a catalytic composition according to any one of claims 1 to 9 or able to be prepared by a method according to any one of claims 10 to 12, for the dehydrogenation of alkanes to alkenes and hydrogen.

14. Use according to claim 13, **characterised in that** the dehydrogenation is carried out a temperature of between 300 and 800°C, at a mass flow rate of the charge in relation the mass of catalyst (pph) of between 1 and 50 h⁻¹, and at a pressure of between 10³ and 10⁶ Pa.

15. Use according to claim 13 and 14, **characterised in that** the alkenes are isobutane and the alkenes isobutene, and **in that** the dehydrogenation is carried out a temperature of between 500 and 550°C and at a pressure of 10⁵, in the presence of an inert gas.

16. Use according to any one of claims 13 to 15, **characterised in that** the dehydrogenation is carried out in the presence of exogenous hydrogen.
